# EUROPEAN PATENT APPLICATION

(11) **EP 4 275 679 A1**
(43) Date of publication of application: **15.11.2023**
(21) Application number: 22172603.7
(22) Date of filing: 10.05.2022
(51) Int. Cl.: A61K 31/122, A61K 31/136, A61P 31/04

(54) **NOVEL ANTIBIOTIC WITH QUORUM SENSING INHIBITOR ACTIVITY**

(71) Applicant: Koninklijke Nederlandse Akademie van Wetenschappen, 1011 JV Amsterdam (NL)
(72) Inventor: Beenker, Wouter Antonius Gerardus, 3552 GV Utrecht (NL); den Hertog, Jeroen, 3997 MH 't Goy (NL)
(74) Representative: Nederlandsch Octrooibureau

(57) **Abstract**

The present invention relates to paecilomycone compound that may be isolated from a fungus, *Aspergillus allahabadii,* which inhibit bacterial quorum sensing, biofilm formation and virulence factor production. Paecilomycone may be used as medicament, in particular as medicament for treating and preventing microbial infections, such as infections by multidrug resistant bacteria, by itself or in combination with other antimicrobial agents.

## Description

### Field of the invention

The present invention relates to the fields of medicine, microbiology and organic chemistry. In particular, the invention relates to a compound obtainable from a fungus that has bacterial quorum sensing inhibitor activity, antimicrobial activity, methods for its production and for its use in treating microbial infections.

### Background of the invention

Bacterial infection is a serious threat to human and animal life. Antibiotics have been developed that counter bacterial infection. The first antibiotic drug, Penicillin, was discovered in 1928 and turned out to be very effective in combating bacterial infections. Penicillin has literally saved millions of human lives. Many more antibiotics have been discovered since. However, resistance is emerging to existing antibiotics and increasing globalization is adding to the rapid spread of bacterial infections. So-called superbugs are emerging that are resistant to virtually all currently known antibiotics. These now form a serious threat to human life. Methicillin-resistant *Staphylococcus aureus,* or MRSA, extended-spectrum beta-lactamase (ESBL)-producing *Enterobacteriaceae*, pathogenic *Streptococcus pneumoniae* are notorious bacterial strains that are resistant to known antimicrobials. In addition, bacterial species, such as *Mycobacterium tuberculosis* are (re-)emerging as human disease-inducing agents as well.

Therefore, there is an increasing need in the art for novel antibiotics, preferably novel antibiotics that are useful for combating infection by bacteria that are resistant to most or all currently known antibiotics. A potential target for alternative treatments is quorum sensing (QS). Via QS, bacteria sense the density of the bacterial population. When the density is high, QS pathways are activated and bacteria start collaborating. QS is involved in pathways that regulate virulence factor production and biofilm formation, described in Bassler, B. L. & Losick, R. Bacterially Speaking. (2006) Cell 125, pp. 237-246. Bacteria in biofilms are less susceptible to immune responses and antibiotic treatments. Hence, inhibition of QS may lead to an increase in susceptibility to antibiotics and faster clearing of bacteria, as described in Bjarnsholt, T. et al. Pseudomonas aeruginosa tolerance to tobramycin, hydrogen peroxide and polymorphonuclear leukocytes is quorum-sensing dependent. (2005) Microbiology 151, pp. 373-383 and Christensen, L. D. et al. Impact of Pseudomonas aeruginosa quorum sensing of biofilm persistence in an in vivo intraperitoneal foreign-body infection model. (2007) Microbiology 153, pp. 2312-2320. In addition, inhibition of virulence factor production may prevent bacteria from damaging the host tissue, as described in Defoirdt, T. Quorum-Sensing Systems as Targets for Antivirulence Therapy. (2018) Trends Microbiol. 26, 313-328. Rather than killing the bacteria, QS inhibition may weaken the bacteria, which makes it possible to fight bacteria with the immune system or in combination with other antibiotics. It is an object of the present invention to provide such a novel antibiotic, methods for its production and for its use in treating bacterial infections.

### Summary of the invention

The present invention concerns a paecilomycone derivative for use in treating or preventing a microbial infection. The invention may also be worded as a method for treating or preventing a microbial infection comprising administering to a subject in need thereof a composition comprising a paecilomycone derivative. The invention may also be worded as the use of a paecilomycone derivative for the manufacture of a medicament for use in treating or preventing a microbial infection. Related to the use according to the first aspect is the use of a paecilomycone derivative for preventing or reducing biofilm formation or bacterial proliferation. The invention further concerns the use of a paecilomycone derivative for inhibiting quorum sensing or biofilm formation.

Surprisingly, the inventors found that the paecilomycone derivatives having a phenalene structure (1) as defined below exhibit a markedly increased quorum sensing inhibitory effect than funalenone, which is structurally closely related to the paecilomycone derivatives, but does not have methoxy group on position C7 but instead on position C2. Paecilomycone derivatives have been described by Lu et al. in J. Agric. Food Chem. 2014, 62, 11917-11923, and funalenone by Inokoshi et al. in J Antibiot. 1999, 52, 1095-1100. The present inventors are the first to demonstrate an antibacterial effect for paecilomycone derivatives.

### Description of the invention

The present invention is centred around paecilomycone derivatives of structure (1), in particular the quorum sensing inhibitory activity thereof. This is reflected in the various aspects of the present invention, which are defined here below as uses, methods and compositions. The skilled person will appreciate that each aspect of the compound is applicable to all aspects of the present invention, just as preferred embodiments of one aspect is equally applicable for a related aspect of the invention.

### The compound

The invention concerns paecilomycone derivatives having a phenalene structure (1):

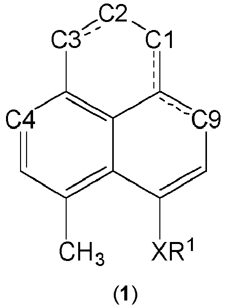

Herein, each of C1, C2, C3, C4 and C9 are individually selected from C=O, C-OH, C-OR², C=S, C-SH, C-SR², C-NH₂ or C-N(R²)₂, wherein the carbon atom is part of the phenalene ring structure. Each R² is individually C₁₋₆ alkyl, C(Y)-C₁₋₆ alkyl, C(Y)-Y-C₁₋₆ alkyl and C(Y)-N(H)₀₋₂(C₁₋₆ alkyl)₀₋₂, wherein each Y is individually O or S. preferably, all Y are O. Herein, the alkyl groups are preferably C₁₋₄ alkyl groups, more preferably C₁₋₂ alkyl groups. Preferably, at least C2, more preferably each of C1, C2, C3, C4 and C9 are individually selected from C=O, C-OH or C-NH₂. It is further preferred that C2 is C=O or C-OH. It is preferred that there are an odd number of C=O moieties, such as 1, 3 or 5, preferably 1 or 3. In a preferred embodiment, one of C1 and C9 is C=O, and/or C2 and C3 are both C=O or are both not C=O.

The substituent on C7 is denoted as XR¹. Herein, R¹ is selected from monocyclic aryl, C₁₋₆ alkyl, C(O)-C₁₋₆ alkyl, C(O)-O-C₁₋₆ alkyl and C(O)-N(H)₀₋₂(C₁₋₆, alkyl)₀₋₂. Preferably, R¹ is C₁₋₄ alkyl, more preferably methyl or ethyl, most preferably R¹ is methyl. X is S or O, preferably X is O. In an especially preferred embodiment, XR¹ = OCH₃.

In a preferred embodiment: C1 is selected from C=O, C-OH or C-NH₂; C2 is C=O or C-OH; C3 is C=O or C-OH; C4 is C-OH; and C9 is C=O or C-OH. Herein, it is preferred that one of C1 and C9 is C=O, and/or C2 and C3 are both C=O or are both not C=O.

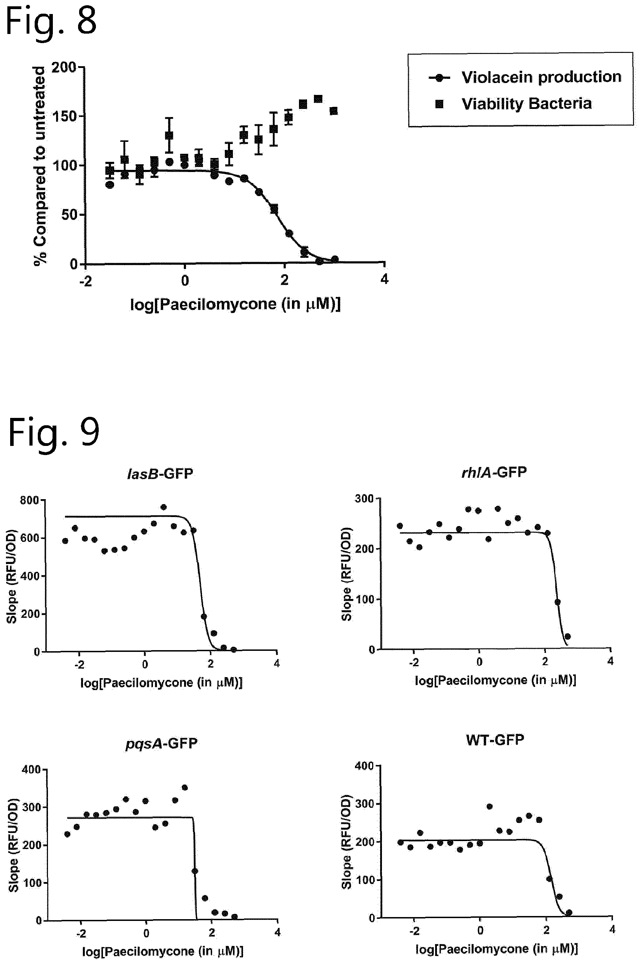

In an especially preferred embodiment, the compound is paecilomycone A, paecilomycone B or paecilomycone C or a mixture thereof. In the context of the present invention, paecilomycone A, paecilomycone B and paecilomycone C are together referred to as "paecilomycone". The structures of these compounds are depicted in Figure 8. In a preferred embodiment, the compound is paecilomycone A, wherein XR¹ = OCH₃; C1 = C2 = C3 = C4 = C-OH; C5 = C=O. In a preferred embodiment, the compound is paecilomycone B, wherein XR¹ = OCH₃; C1 = C2 = C3 = C=O, C4 = C5 = C-OH. In a preferred embodiment, the compound is paecilomycone C, wherein XR¹ = OCH₃; C1 = C-NH₂; C2 = C3 = C4 = C-OH, C5 = C=O. In a most preferred embodiment, the compound is paecilomycone A.

Also envisioned in the present invention are salts, esters and prodrugs of the compounds according to the invention, preferably pharmaceutically acceptable salts, esters and prodrugs of the compounds and more preferably pharmaceutically acceptable salts of the compounds.

The compound may also be present in a mixture of several compounds having a phenalene structure (1). Such a mixture of compounds preferably comprises at least one of paecilomycone A, paecilomycone B and paecilomycone C, preferably a mixture of two or even all three thereof. Most preferably, the mixture comprises at least paecilomycone A, preferably in combination with one or both of paecilomycone B and paecilomycone C.

Preferably, the compound according to the invention is in isolated form. When the compound is a mixture of compounds having a phenalene structure (1), they may be isolated together as a mixture. The compounds according to the invention may be obtained by synthesis or by isolation from natural sources, e.g. as (secondary) metabolite from *Aspergillus allahabadii* (available as CBS 164.63).

The compound of the invention has quorum sensing inhibitor activity. Quorum sensing inhibitor activity can be assessed by the person skilled in the art, preferably by a quorum sensing assay as exemplified herein. *Chromobacterium violaceum* bacteria produce violacein, a purple compound when quorum sensing is activated. C. *violaceum* bacteria are grown o/n at 27°C in TSB medium (3 % w/v tryptic soy broth in H₂O). The bacteria are diluted 1000x in TSB medium and grown until OD600=0.5-0.7. The bacteria are resuspended in TSB medium containing compound and incubated at 27°C for 20 h on an orbital shaker. The medium is aspirated and the precipitated violacein is dissolved in 96 % ethanol. To avoid interference due to cell turbidity, the samples are centrifuged another time and dissolved violacein is transferred to a new plate. The amount of violacein is assessed spectrophotometrically by assessment of the OD at 562 nm. If the OD₅₆₂ of compound treated samples is less than 80 % of the OD₅₆₂ of control bacteria, the sample is scored as inhibitory. To ascertain that reduced OD₅₆₂ signal is due to inhibition of quorum sensing and not merely due to reduced viability of bacteria, a viability assay may be done in parallel to the quorum sensing assay. To assess viability of bacteria, C. *violaceum* bacteria are grown o/n at 27°C in TSB medium. The bacteria are diluted 1000x in TSB medium and grown until OD600=0.5-0.7. The bacteria are resuspended in TSB medium containing compound and incubated at 27°C for 20 h. Bacteria are collected by centrifugation for 10 min at 3000 rpm. Supernatant is aspirated and resazurin (150 µg/mL in physiological salt solution) is added. Plates are incubated for another 45 min at 27°C and fluorescence is measured by excitation at 540 nm and emission at 590 nm. If the fluorescent signal is less than 70 % of the fluorescent signal of control untreated bacteria, the sample is scored as having an effect on viability of the bacteria.

The compound preferably has quorum sensing inhibitor activity in Pseudomonas aeruginosa (strain PAO1). Quorum sensing activity in PAO1 cells can be assessed by a the person skilled in the art, preferably by a quorum sensing reporter assay as exemplified herein. PAO1 reporters express green fluorescent protein under the control of the *lasB* promoter, the *rhlA* promoter, the *pqsA* promoter or a constitutive promoter, *P*_{*A1*/*04*/}*₀₃.* Reporter bacteria are inoculated in AB minimal media supplemented with 0.5 % glucose and 0.5 % casamino acids. Cultures are diluted in physiological salt solution to an OD450 = 0.1-0.2 before addition to AB medium with a range of concentrations of compound. The GFP fluorescence (excitation 485 nm, emission 535 nm) and absorbance (600 nm) are determined every 15 min for 15 h at 34°C. IC50 values are calculated based on the maximum slope of GFP/OD. If the IC50 on reporter lines is less than half of the IC50 on control bacteria, the compound is scored as quorum sensing inhibitor.

The compound of the invention may have antimicrobial or antibiotic activity, more preferably antibacterial activity. Antimicrobial or antibacterial activity can be assessed by a suitable assay known by the person skilled in the art, preferably by a growth inhibition assay as exemplified herein. Preferably growth inhibition is assessed by culturing bacteria that are cultured in the presence or absence of the antibacterial compound o/n at 37°C in Mueller-Hinton Broth (MHB). The read-out of the assay is by eye, if the bacteria have visibly grown, the sample is scored as non-inhibitory. If the broth is completely clear, the sample is scored as inhibitory.

The antimicrobial effect of the compound of the invention may also be exemplified by a growth kinetic assay, in which the effect of the compound of interest is assessed in time on growth of bacterial isolates in liquid medium, as known by the person skilled in the art. In brief, bacteria are inoculated in AB minimal medium supplemented with 0.5 % glucose and 0.5 % casamino acids or King's A medium and incubated at 37°C o/n. The o/n cultures are diluted in physiological salt solution until an OD₆₀₀=0.1-0.2. Diluted cultures (150 µL) are transferred to a honeycomb microplate containing 150 µL AB minimal medium supplemented with 0.5 % glucose and 0.5 % casamino acids or King's A medium, with or without antimicrobial agent, rendering a total volume of 300µL. The absorbance at 600 nm is measured every 15 min for 24 h at 34°C on a Bioscreen C (Growth Curves Ab Ltd). A compound is understood herein as having antibacterial activity if the slope of the growth curve is reduced or there is a lag time in bacterial growth.

The compound of the invention may have antibacterial activity against Gram-positive bacteria or against Gram-negative bacteria, or against both. In one embodiment, the compound of the invention has antibacterial activity against Gram-positive bacteria, preferably bacteria that belong to a genus selected from the group consisting of *Streptococcus*, *Staphylococcus*, *Corynebacterium*, *Listeria*, *Bacillus*, *Enterococcus* and *Clostridium,* or, more specifically, bacteria that belong to a species selected from the group consisting of *Streptococcus agalactiae, Streptococcus pneumonia, Streptococcus pyogenes, Staphylococcus aureus, Staphylococcus epidermidis, Staphylococcus saprophyticus, Corynebacterium diphtheria, Listeria monocytogenes, Enterococcus faecalis, Enterococcus faecium, Bacillus anthracis, Bacillus cereus*, *Clostridium botulinum*, *Clostridium difficile*, *Clostridium perfringens*, and *Clostridium tetani* as assessed in an assay as defined herein. Even more preferably, the compound of the invention has antibacterial activity against the strains *Staphylococcus epidermidis* (08A1071), Methicillin-sensitive *Staphylococcus aureus* 476 (S0101) (available from ATCC BA-1721), Methicillin-resistant *Staphylococcus aureus* (MRSA) ST8:USA300 (S1474) (available from NARSA collection NRS482), *Enterococcus faecium* (15A623), *Enterococcus faecium* (16D030), preferably against at least 2, 3, 4, 5 or all of these strains, as assessed in an assay as defined herein. Most preferably, the compound of the invention has antibacterial activity against a methicillin-resistant *Staphylococcus,* preferably a methicillin-resistant *Staphylococcus aureus.* Even more preferably, the compound of the invention has antibacterial activity against methicillin-resistant *Staphylococcus aureus strain* ST8:USA300 (S1474), as assessed in an assay as defined herein.

Alternatively or additionally, the compound of the invention has antibacterial activity against Gram-negative bacteria preferably bacteria that belong to a genus selected from the group consisting of *Acinetobacter, Actinobacillus, Bordetella, Brucella, Campylobacter, Cyanobacteria, Enterobacter, Erwinia, Escherichia coli, Franciscella, Helicobacter, Hemophilus, Klebsiella, Legionella, Moraxella, Neisseria, Pasteurella, Proteus, Pseudomonas, Salmonella, Serratia, Shigella, Treponema, Vibrio* and *Yersinia*, or, more specifically, bacteria that belong to the species selected from *Klebsiella pneumoniae* (MR44), *Acinetobacter calcoaceticus* (15A600), *Pseudomonas aeruginosa*, *Stenotrophomonas maltophilia*, *Klebsiella pneumonia*, *Klebsiella pneumonia*, *Klebsiella pneumonia*, *Escherichia coli*, *Escherichia coli* and *Escherichia coli*, as assessed in an assay as defined herein. Even more preferably, the compound of the invention has antibacterial activity against the strains *Escherichiae coli* (available from ATCC, number 25922), *Klebsiella pneumonia* (MR44), *Pseudomonas aeruginosa* (available from ATCC, number 27853), *Pseudomonas aeruginosa* (PAO1), *Acinetobacter baumanii* (12-5112), *Acinetobacter calcoaceticus* (15A600, available from NCCB, number 100514), preferably against at least 2, 3, 4, 5, 6 or all of these strains, as assessed in an assay as defined herein.

Preferably, a compound is understood herein as having antibacterial activity if a sample comprising at most 250 µM, 125 µM, 63 µM, 31 µM, 16 µM, 8 µM, 4 µM, 2 µM or preferably at most 1 µM of the compound is scored in an assay as defined herein when using a bacterium as defined herein. More preferably, a compound is understood herein as having antibacterial activity if the compound has at least 30%, 40%, 50%, 60%, 70%, 80%, 90% or preferably 100% of the activity of paecilomycone, as assessed in an assay as defined herein using a bacterium as defined herein.

The compound according to the present invention can be prepared synthetically or can be isolated from a natural source. In one embodiment, the method for producing the compound preferably comprises the steps of (a) culturing a fungus of the fungal Family of *Aspergillus* in a medium, and preferably under conditions, conducive to the production of the compound, and (b) optionally, recovery of the compound. Preferably, the fungus of the fungal Family of *Aspergillus* is a fungus that produces a compound according to the invention. A fungus producing a compound according to the invention is herein understood as a fungus which, when grown in a standard medium for culturing fungi (e.g. potato dextrose broth (PDB)+ 2 % glucose), produces in the medium a compound with one or more of the characteristics: (a) the compound has antimicrobial activity in an assay as herein defined above; (b) the compound can be extracted from the medium using ethyl acetate; (c) the compound has a molecular mass of around 289, 287 or 286; (d) the compound has a UV maximal absorbance 213, 254 or 207 nm; and other characteristic defined herein or determined in the Examples herein.

Preferably, the fungus is a fungus that belong to the genus *Aspergillus,* more preferably, the fungus is a fungus of the species *Aspergillus allahabadii,* and most preferably the fungus is the *Aspergillus allahabadii* strain CBS 164.63, or strains derived from these fungi, e.g. by genetic modification and/or mutagenesis and selection.

The medium for culturing the fungus is a medium comprising at least a suitable carbon source and further comprising further nutrients and ingredient that support growth of the fungus. Suitable media for fermentation of fungi are well known in the art per se. Suitable carbon sources include e.g. glucose, syrups, dextrin, sucrose, starch, molasses and the like can be used. The medium will further usually include a nitrogen source such as e.g. soybean flour, wheat germ, corn-steep liquor, cottonseed cake, meat extract, peptone, yeast extract, ammonium sulfate, sodium nitrate, urea, and the like. Further medium components may be inorganic salts capable of yielding sodium, potassium, calcium, magnesium, cobalt, chloric, phosphoric, sulfuric and like ions.

In step (a) of the method, the fungus preferably ferments (at least part of) the carbon source to the compound according to the invention. Preferably the fungus is cultured in step (a) at a temperature less than 30 °C. More preferably, the fermentation (in step a) is performed at a temperature of no more than 29, 28, 27 or 26 °C and which is at least 20, 21, 22, 23 or 24 °C Most preferably the temperature is around 25 °C. The fermentation preferably is an aerobic fermentation, especially a submerged aerobic fermentation is preferred.

The process for producing the compound further preferably comprises a step (b) wherein the compound is recovered, purified and/or isolated. The compound is preferably recovered from medium by solvent extraction, optionally after removal of biomass from the medium. A skilled person will know suitable organic solvents for extraction of the compound from the medium and/or from the separated mycelium, such as e.g. ethyl acetate, chloroform, dichloromethane, ether, ethyl acetate, butyl acetate, methanol, ethanol, propanol, butanol or combinations thereof. A preferred solvent for solvent extraction is ethyl acetate. After concentration of the extracts the compound of the invention can be further purified and isolated e.g. by using usual chromatographic adsorbents or carrier materials.

### The pharmaceutical composition

The compound according to the invention is typically comprised in a pharmaceutical composition, wherein the compound is the active ingredient. An active ingredient is to be understood herein as an ingredient that has quorum sensing inhibitory activity.

The pharmaceutical composition preferably further comprises a pharmaceutically acceptable carrier, excipient and/or vehicle, in addition to the active ingredient. Thus, the compounds according to the invention can be formulated as pharmaceutical or compositions by formulation with additives such as pharmaceutically or physiologically acceptable excipients, carriers, and vehicles. Suitable pharmaceutically or physiologically acceptable excipients, carriers and vehicles include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-P-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences, " Mack Pub. Co. , New Jersey (1991), and "Remington: The Science and Practice of Pharmacy, " Lippincott Williams & Wilkins, Philadelphia, 20th edition (2003) and 21" edition (2005), incorporated herein by reference.

A pharmaceutical or composition can comprise a unit dose formulation, where the unit dose is a dose sufficient to have a therapeutic effect. The unit dose may be sufficient as a single dose to have a therapeutic effect. Alternatively, the unit dose may be a dose administered periodically in a course of treatment of an infectious disease.

Pharmaceutical compositions containing the compounds of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, liposomal encapsulates, and the like, as well as combinations of any two or more thereof.

Time-release or controlled release delivery systems may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed. , Marcel Dekker, Inc. , New York 1992. The matrix may be, for example, a biodegradable material that can degrade spontaneously in situ and in vivo for, example, by hydrolysis or enzymatic cleavage, e.g. , by proteases. The delivery system may be, for example, a naturally occurring or synthetic polymer or copolymer, for example in the form of a hydrogel. Exemplary polymers with cleavable linkages include polyesters, polyorthoesters, polyanhydrides, polysaccharides, poly(phosphoesters), polyamides, polyurethanes, poly(imidocarbonates) and poly(phosphazenes).

The compounds of the invention may be administered enterally, orally, parenterally, sublingually, by inhalation (e. g. as mists or sprays), rectally, or topically in dosage unit formulations containing conventional nontoxic pharmaceutically or physiologically acceptable carriers, adjuvants, and vehicles as desired. For example, suitable modes of administration include oral, subcutaneous, transdermal, transmucosal, iontophoretic, intravenous, intraarterial, intramuscular, intraperitoneal, intranasal (e. g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intrasternal injection, or infusion techniques. The compounds are mixed with pharmaceutically acceptable carriers, adjuvants, and vehicles appropriate for the desired route of administration. Oral administration is a preferred route of administration, and formulations suitable for oral administration are preferred formulations. The compounds described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. The compounds can also be administered as prodrugs, where the prodrug undergoes transformation in the treated subject to a form which is therapeutically effective. Additional methods of administration are known in the art.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at room temperature but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N. Y., p. 33 et seq (1976).

The compound according to the invention is administered in a therapeutically effective amount, which will vary depending upon a variety of factors including the activity of the specific pharmaceutical composition employed; the metabolic stability and length of action of the composition; the age, body weight, general health, sex, and diet of the patient; the mode and time of administration; the rate of excretion; the drug combination; the severity of the particular disorder or condition; and the subject undergoing therapy. Generally, a therapeutically effective daily dose is from about 0.001 mg/kg to about 100 mg/kg, preferably, from about 0.01 mg/kg to about 50 mg/kg, more preferably from about 1 mg/kg to about 25 mg/kg.

For veterinary use, the dose is generally in the range of from 1 to 200 mg, and preferably from 5 to 100 mg, per kg of body weight per day while varying depending on the purpose of administration (for therapy or for prevention), the kind and the size of the animal, the kind of the pathogenic organisms, and severity of symptom.

A pharmaceutical composition of the invention intended e.g. for parenteral or oral administration should contain an amount of a compound of the invention such that a suitable dosage will be obtained. Typically, the amount of the compound is at least 0.01% of a pharmaceutical composition of the invention. When intended for oral administration, this amount may be varied to be between 0.1 and about 70% of the weight of the composition. Preferred oral pharmaceutical compositions contain between about 4% and about 50% of the compound of the invention. Preferred pharmaceutical compositions and preparations according to the present invention are prepared so that a parenteral dosage unit contains between 0.01 to 10% by weight of the compound of the invention prior to dilution.

### Application

The compound according to the invention is suitable for use as a medicament, in particular in treating or preventing a microbial infection. The medicament can be a medicament for both human and veterinary purposes. The invention thus also relates to a compound according to the invention for use in treating or preventing a microbial infection. Preferably, the compound is for use in treating or preventing a microbial infection in a human or animal subject. The microbial infection preferably is a bacterial infection. In an alternative embodiment, the compound according to the invention is used in cosmetic or non-therapeutic method for treating or preventing microbial infections and/or for disinfection human or animal subjects and body parts thereof. Accordingly, the above described pharmaceutical compositions comprising a compound according to the invention as active ingredient can thus also be or used as cosmetic compositions or disinfectants.

In one embodiment, the compound according to the invention is used for treating or preventing an infection by a multidrug resistant bacterium. A multidrug resistant bacterium is herein understood as a bacterial strain that is resistant to more than one type of antibiotic and that are therefore more difficult, if not impossible to treat. Notorious examples of multidrug resistant bacteria include e.g. methicillin-resistant *Staphylococcus aureus* (MRSA) and extended-spectrum beta-lactamase (ESBL)-producing *Enterobacteriaceae.*

The compound according to the invention may be active against Gram-positive bacteria, Gram-negative bacteria or both. In one embodiment, the compound according to the invention is used for treating or preventing an infection by a Gram-positive bacterium, in particular a pathogenic Gram-positive bacterium. Infections by Gram-positive bacteria that can be treated and/or prevented using a compound or composition according to the invention include infections by bacteria that belong to a genus selected from *Streptococcus*, *Staphylococcus*, *Corynebacterium*, *Listeria*, *Bacillus*, *Enterococcus* and *Clostridium,* or, more specifically, bacteria that belong to the species selected from *Streptococcus agalactiae*, *Streptococcus pneumonia, Streptococcus pyogenes, Staphylococcus aureus, Staphylococcus epidermidis, Staphylococcus saprophyticus, Corynebacterium diphtheria, Listeria monocytogenes, Enterococcus faecalis, Enterococcus faecium, Bacillus anthracis, Bacillus cereus*, *Clostridium botulinum*, *Clostridium difficile*, *Clostridium perfringens*, and *Clostridium tetani.* Good results are obtained when the compound or composition according to the invention is used for treating and/or preventing an infection by a methicillin-resistant *Staphylococcus,* preferably a methicillin-resistant *Staphylococcus aureus.*

In an alternative embodiment, the compound according to the invention is used for treating or preventing an infection by a Gram-negative bacterium, in a particular a pathogenic Gram-negative bacterium. Infections by Gram-negative bacteria that can be treated and/or prevented using a compound or composition according to the invention include infections by bacteria that belong to a genus selected from *Acinetobacter, Actinobacillus, Bordetella, Brucella, Campylobacter, Cyanobacteria, Enterobacter, Erwinia, Escherichia coli, Franciscella, Helicobacter, Hemophilus, Klebsiella, Legionella, Moraxella, Neisseria, Pasteurella, Proteus, Pseudomonas, Salmonella, Serratia, Shigella, Treponema, Vibrio* and *Yersinia,* or, more specifically, bacteria that belong to the species selected from *Klebsiella pneumoniae* (MR44), *Acinetobacter calcoaceticus* (15A600), *Pseudomonas aeruginosa*, *Stenotrophomonas maltophilia, Klebsiella pneumonia, Klebsiella pneumonia, Klebsiella pneumonia, Escherichia coli*, *Escherichia coli* and *Escherichia coli.*

In a preferred embodiment, the compound according to the invention is used for treating and/or preventing an infection by a *Mycobacterium* or a *Mycoplasma*, such as e.g. *Mycobacterium leprae*, *Mycobacterium tuberculosis*, *Mycobacterium ulcerans* and *Mycoplasma pneumonia.*

Worded differently the invention pertains to a method for treating or preventing microbial infection, the method comprising administering to a subject in need thereof an effective amount of the compound according to the invention. Typically, the subject suffers from a microbial infectious disease. The subject can be human or animal. The microbial infection can be an infection by a bacterium as herein defined above. The invention according to this aspect can also be worded as the use of the compound according to the invention for the manufacture of a medicament for use in treating or preventing a microbial infection.

A compound according to the invention can be used in methods for the treatment or prevention of an microbial infection or disease selected from, for example, bacterial infection of wounds including surgical wounds, lung infections (e.g. tuberculosis, cystic fibrosis), skin infections, and systemic bacterial infections. For instance, diseases which can be treated or prevented by the antimicrobial compounds of the present invention include, but are not limited to, anthrax, cystic fibrosis, food poisoning, folliculitis, furuncle, carbuncle, erysipelas, phlegmon, lymphangitis/lymphadenitis, felon, subcutaneous abscess, spiradenitis, acne agminata, infectious atheroma, perianal abscess, masitadenitis, superficial secondary infections after trauma, burn or surgery trauma, pharyngolaryngitis, acute bronchitis, tonsillitis, chronic bronchitis, bronchiectasis, diffuse panbronchiolitis, secondary infections of chronic respiratory diseases, pneumonia, pyelonephritis, cystitis, prostatitis, epididymitis, urethritis, cholecystitis, cholangitis, bacillary dysentery, enteritis, adnexitis, intrauterine infections, bartholinitis, blepharitis, hordeolum, dacryocystitis, tarsadenitis, keratohelcosis, otitis media, sinusitis, paradentosis, pericoronitis, gnathitis, peritonitis, endocarditis, septicemia, meningitis, and skin infections.

In a preferred embodiment, the treatment or prevention according to the invention involves a combination treatment, wherein the compound according to the invention is combined with a second (or further) active agent, typically an antibiotic agent. Such a combination treatment with two (or more) active components is well-known in the art, and can be executed in any way known to the skilled person.

Related to the above-defined use, the invention also concerns the use of the compound according to the invention for preventing or reducing biofilm formation. Worded differently, the invention concerns a method for preventing or reducing biofilm formation, comprising contacting the compound according to the invention to the subject or item where biofilm formation occurs or might occur. This use or method may be medical or non-medical. The quorum sensing inhibitory activity of the compound according to the invention, as found by the inventors, negatively affects bacterial biofilm formation, such that such biofilm formation is prevented or reduced.

Related to the above-defined use, the invention also concerns the use of the compound according to the invention for preventing or reducing bacterial proliferation. Worded differently, the invention concerns a method for preventing or reducing bacterial proliferation, comprising contacting the compound according to the invention to the subject or item where bacterial proliferation occurs or might occur. This use or method may be medical or non-medical. The quorum sensing inhibitory activity of the compound according to the invention, as found by the inventors, negatively affects bacterial proliferation, such that such bacterial proliferation is prevented or reduced.

Related to the above-defined use, the invention also concerns the use of the compound according to the invention for inhibiting bacterial quorum sensing, bacterial biofilm formation and virulence factor production. Worded differently, the invention concerns a method for inhibiting bacterial quorum sensing, bacterial biofilm formation and virulence factor production, comprising contacting the compound according to the invention to the subject or item in need thereof. This use or method may be medical or non-medical. The quorum sensing inhibitory activity of the compound according to the invention, as found by the inventors, negatively affects bacterial quorum sensing, bacterial biofilm formation and virulence factor production, such that it is prevented or reduced.

Related to the above-defined use, the invention also concerns the use the compound according to the invention as an antimicrobial agent, preferably an antibacterial agent. This use may be medical or non-medical. The quorum sensing inhibitory activity of the compound according to the invention, as found by the inventors, negatively affects bacterial growth rates, such that the compound has an antimicrobial effect.

The invention thus further relates to an antimicrobial composition, preferably a disinfectant, comprising a compound according to the invention as active ingredient. A disinfectant is an antimicrobial agent specific for use on inanimate objects. The antimicrobial composition is particularly useful for reducing or preventing biofilm formation such as found in processing and/or medical equipment. The antimicrobial composition or disinfectant of the invention may be in the liquid, solid or semi-liquid or semi-solid form. This aspect of the invention may also be worded as the use of the compound of the invention in an antimicrobial composition, preferably a disinfectant, wherein the use is as antimicrobial agent. Preferably said antimicrobial composition or disinfectant is for controlling a Gram-positive bacterium, a Gram-negative bacteria or both. In one embodiment, the antimicrobial composition is used for controlling a Gram-positive bacterium, in a particular a Gram-positive bacterium belonging to a genus selected from *Streptococcus*, *Staphylococcus*, *Corynebacterium*, *Listeria*, *Bacillus*, *Enterococcus* and *Clostridium,* or, more specifically, to the species selected from *Streptococcus agalactiae, Streptococcus pneumonia, Streptococcus pyogenes, Staphylococcus aureus, Staphylococcus epidermidis, Staphylococcus saprophyticus, Corynebacterium diphtheria, Listeria monocytogenes, Enterococcus faecalis, Enterococcus faecium, Bacillus anthracis, Bacillus cereus, Clostridium botulinum, Clostridium difficile, Clostridium perfringens*, and *Clostridium tetani.* Most preferably, the methicillin-resistant *Staphylococcus,* preferably a methicillin-resistant *Staphylococcus aureus.*

Preferably, the antimicrobial composition or the pharmaceutical composition according to the invention further comprises a pharmaceutical acceptable carrier and/or an additional active ingredient selected from the group consisting of a bacteriophage, a bacteriostatic agent, a bactericide agent, an antibiotic, a surfactant and/or an enzyme. The antibiotic can be any antibiotic known in the art including antibiotics and chemotherapeutic agents. The enzyme includes but is not limited to enzymes that aid in breaking up biofims (e.g. biofilms found in processing and/or medical equipment) such as but not limited to polysaccharide depolymerise enzymes and protease. The surfactant is useful for wetting the surface so that the active ingredient of the present invention, including the compound of the present invention, is properly distributed over the various surfaces, and to solubilise and remove dirt so that the microbes, preferably bacteria are accessible to the active ingredient. Suitable surfactants include but are not limited to polysorbate (tween) 80, 20 and 81 and Dobanols (Shell Chemical Co. RTM). An antimicrobial composition or disinfectant of the present invention may further comprise surface disinfectants known in the art such as but not limited to benzoic acid and PBT, preferably surface disinfectants with which a compound of the first aspect of the present invention is compatible.

Preferably, the antimicrobial composition is used in a method for controlling microbial contamination in processing equipment or medical equipment. In this method, the antimicrobial composition is contacted with processing equipment or medical equipment. Preferably, the method of controlling microbial contamination includes the reduction of counts of bacteria, typically pathogenic bacteria, the prevention of their growth in the first place, and/or the prevention of biofilm formation on processing or medical equipment. Preferably, said method is of controlling for cleaning and sterilizing medical equipment. Herein, medical equipment may refer to fiberscopes, like gastrocameras, peritoneoscopes, thoracoscopes and arthoroscopes, and medical supplies, like catheters and tubes that have long ducts or hollow portions and tend to be repetitively employed by being introduced into human bodies. A method of the present invention encompasses the application of the compound on various physical sites on processing or medical equipment, by a number of means including, but not limited to, admixing, spraying or directly applying said compound according to the first aspect or a compositions according to the sixth aspect of the present invention. Optionally, the method of the present invention can be combined with any sterilization method or disinfectant known in the art such as ultrasonic cleaning, irradiation or thermal sterilization, by immersing the equipment in a disinfectant solution such as ethanol, ammonium, iodine and/or aldehyde disinfectant, or by using gas sterilization by retaining the device in a closed atmosphere such as formaline gas or ethylene oxide gas.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there be one and only one of the elements. The indefinite article "a" or "an" thus usually means "at least one".

All patent and literature references cited in the present specification are hereby incorporated by reference in their entirety.

### Description of the figures

Figure 1: Preparative HPLC chromatogram (x-axis: time in min): Fractionation of extract with TFA as modifier. Black box is the active fraction.
Figure 2: Preparative HPLC chromatogram (x-axis: time in min): Subsequent fractionation of the active fraction with NH₄OAC as modifier . The black box is the active fraction.
Figure 3: Analytical HPLC chromatogram of active fraction in TFA (x-axis: time in min). Showing besides the largest peak (paecilomycone A), also two additional peaks (paecilomycone B and paecilomycone C). UV-VIS spectra of corresponding peaks are included (x-axis: wavelength in nm).
Figure 4: LC-MS data of active compound (x-axis = m/z). (Top) ESI(+) spectrum, showing a peak at 289 [M+H], (Bottom) ESI(-) spectrum showing a peak at 287 [M-H].
Figure 5: LC-MS data of peak corresponding to paecilomycone B (x-axis = m/z). (Top) ESI(+) spectrum, showing a peak at 287 [M+H], (Bottom) ESI(-) spectrum showing a peak at 285 [M-H].
Figure 6: LC-MS data of peak corresponding to paecilomycone C (x-axis = m/z). (Top) ESI(+) spectrum, showing a peak at 288 [M+H], (Bottom) ESI(-) spectrum showing a peak at 286 [M-H].
Figure 7: Chemical structures of the paecilomycones.
Figure 8: Quorum sensing inhibition in C. *violaceum* after treatment with paecilomycone. Experiment was done in triplicates, error bars represent the mean ± SEM.
Figure 9: Quorum sensing inhibition in *Pseudomonas aeruginosa* (PAO1) reporters after treatment with paecilomycone. Calculated IC₅₀ values are 49.8 µM (*lasB*-GFP), 233.8 µM (rhIA-GFP), 31.5 µM (*pqsA*-GFP), and 143.2 µM (WT-GFP). Experiments were done in triplicates. Plotted is the maximum slope of the average RFU/OD.
Figure 10: Growth curves of *Pseudomonas aeruginosa* in AB minimal medium after treatment with various concentrations of paecilomycone (gray curves) versus untreated (black curves). Experiment was done in triplicates, error bars represent the mean ± SEM.

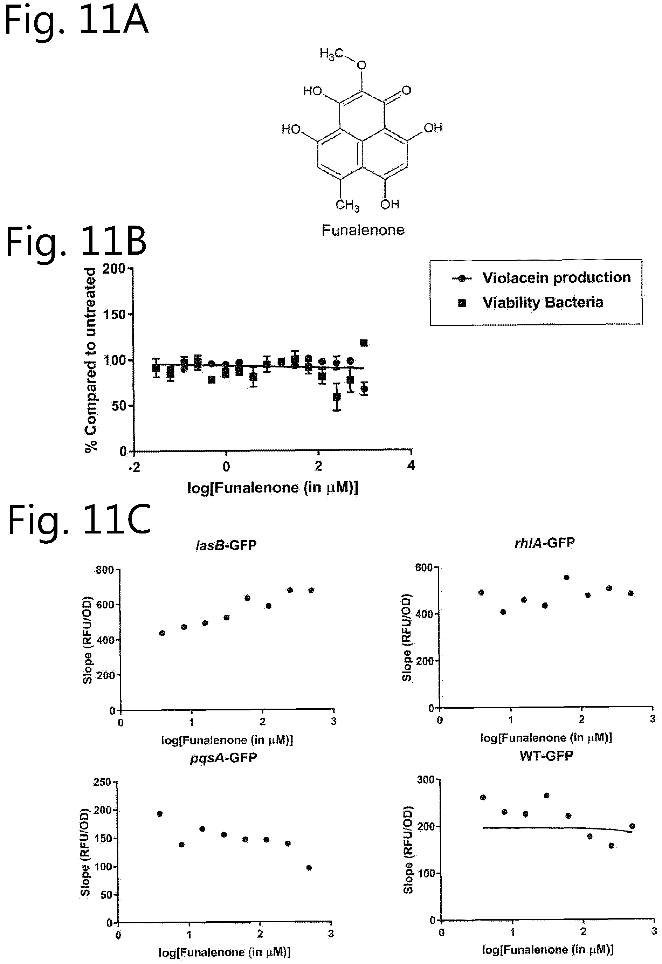

Figure 11: Quorum sensing is not inhibited in response to closely related funalenone. (A) The chemical structure of funalenone, which is very similar to paecilomycone, but differs with respect to the position of the methoxy group. (B) Quorum sensing activity of C. *violaceum* and assessment of bacterial viability in response to treatment with funalenone. Experiment was done in triplicates, error bars represent the mean ± SEM. (C) Quorum sensing inhibition in *Pseudomonas aeruginosa* (PAO1) reporters after treatment with funalenone. Experiments were done in triplicates. Plotted is the maximum slope of the average RFU/OD.
Figure 12: Biofilm formation inhibition in PAO1 after treatment with paecilomycone. Experiments were done with biological triplicates containing technical triplicates. Averages of the biological replicates were used for analysis. Error bars represent the mean ± SEM. A one-way ANOVA, corrected for multiple comparisons with Dunnett's test was performed to determine statistical significance. Values are compared to DMSO treated controls (**, P<0.005; ***, P<0.001).
Figure 13: Inhibition of pyocyanin synthesis in PAO1 after treatment with paecilomycone. Plot represents representable experiment performed in triplicates. Error bars represent the mean ± SEM. A one-way ANOVA, corrected for multiple comparisons with Dunnett's test was performed to determine statistical significance. Values are compared to DMSO treated controls (***, P<0.001; ****, P<0.0001).
Figure 14: Growth curves of *Pseudomonas aeruginosa* in King's A medium after treatment with various concentrations of paecilomycone (gray curve) versus untreated (black curve). Experiment was done in triplicates, error bars represent the mean ± SEM.
Figure 15: Activation of rhamnolipid synthesis after treatment with paecilomycone. Experiment were done with biological triplicates containing technical triplicates. Averages of the biological replicates were used for analysis. Error bars represent the mean ± SEM. A one-way ANOVA, corrected for multiple comparisons with Dunnett's test was performed to determine statistical significance. Values are compared to DMSO treated controls (*, P<0.05).
Figure 16: Antibiotic susceptibility microdilution test with various concentrations of paecilomycone in MHB medium in various Gram-negative and Gram-positive bacterial strains. Bacterial growth is measured at 595 nm and 620 nm every 15 min for 24 h.
Figure 17: Antibiotic susceptibility microdilution test with various concentrations of paecilomycone in AB minimal medium in various Gram-negative bacteria. Bacterial growth is measured at 595 nm and 620 nm every 15 min for 24 h.

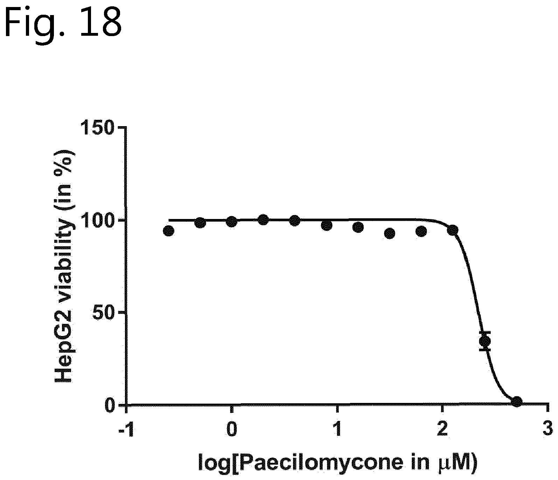

Figure 18: Cell viability assay (resazurin assay) on HepG2 cells after treatment with paecilomycone. Experiment was done in triplicates, error bars represent the mean ± SEM.

### Examples

The following examples are offered for illustrative purposes only, and are not intended to limit the scope of the present invention in any way.

### Growth of fungi

The fungus *Aspergillus allahabadii* strain CBS 164.63, (obtainable from Westerdijk Fungal Biodiversity Institute, Utrecht, The Netherlands; www.wi.knaw.nl) was cultured on a malt-extract agar (MEA) plate for 7 days at 25°C. The fungus was subsequently inoculated in 3 flasks containing 50 ml potato dextrose broth (PDB)+2 % glucose. Each flask was incubated at a different temperature - 15°C, 25°C or 25°C with 100 rpm orbital shaking - for 7 days. After 7 days, the fungal media were filter-sterilized (0.22µm pore filter). The filtrate was tested on C. *violaceum* as follows.

### Quorum sensing assay

C. *violaceum* bacteria were grown o/n in tryptic soy broth at 27°C. The bacteria were diluted in TSB and grown until OD600=0.5-0.7. Bacteria were diluted 1:1000 and 50 µL bacterial suspension was transferred to 96-well plates containing 50 µL fungal filtrate, extract or purified compound in a range of concentrations. Purified compounds of formula 1, 2 and 3 were tested in triplicates by 2-fold dilutions. Plates were incubated for 20 h at 27°C while shaking at 180 rpm. Plates were centrifuged for 10 min at 3000 rpm to precipitate the violacein. Supernatant was aspirated and the pellet was resuspended in 200 µL 96 % ethanol. The plates were centrifuged again for 10 min at 3000 rpm and 100 µL of the supernatant containing the violacein was transferred to a new 96-well plate and the violacein production was quantified by measuring the OD₅₆₂ on an ASYS expert plus microplate reader. To measure the viability of bacteria, plates grown in parallel with the plates described above, were centrifuged for 10 min at 3000 rpm. Supernatant was aspirated and resazurin (150 µg/mL in physiological salt solution) was added. Plates were incubated for another 45 min at 27°C before fluorescence was measured on a PHERAstar microplate reader (BMG Labtech) using 540 nm excitation and 590 nm emission wavelength.

### Purification of compounds with quorum sensing inhibitor activity

Based on the first results, 60 jars of 50ml PDB + 2 % glucose were inoculated with fungus *Aspergillus allahabadii* strain CBS 164.63 and incubated at 25°C with 100 rpm orbital shaking for 7 days. The medium was filter sterilized as described, and extracted 3x with EtOAc. The EtOAc was evaporated and the residue was dissolved in 2 ml DMSO. The extract was then fractionated using a C18 Reprosil column on a Prep. HPLC (see Table 5 for apparatus information) with UV-detection between 214 nm and 254 nm. The buffers used are described in Table 1, the buffer B gradient runs as described in Table 2.

**Table 1: HPLC buffers**

| **Buffer A** | **Buffer B** |
|---|---|
| 100% MQ | 100% Acetonitrile |
| 0.1% Trifluoroacetic Acid | 0.1% Trifluoroacetic Acid |

**Table 2: HPLC gradient**

| **Time (min.)** | **Percentage buffer B** |
|---|---|
| 0-5 | 5 |
| 5-45 | 5-95 |
| 45-50 | 95 |
| 50-55 | 95-5 |
| 55-60 | 5 |

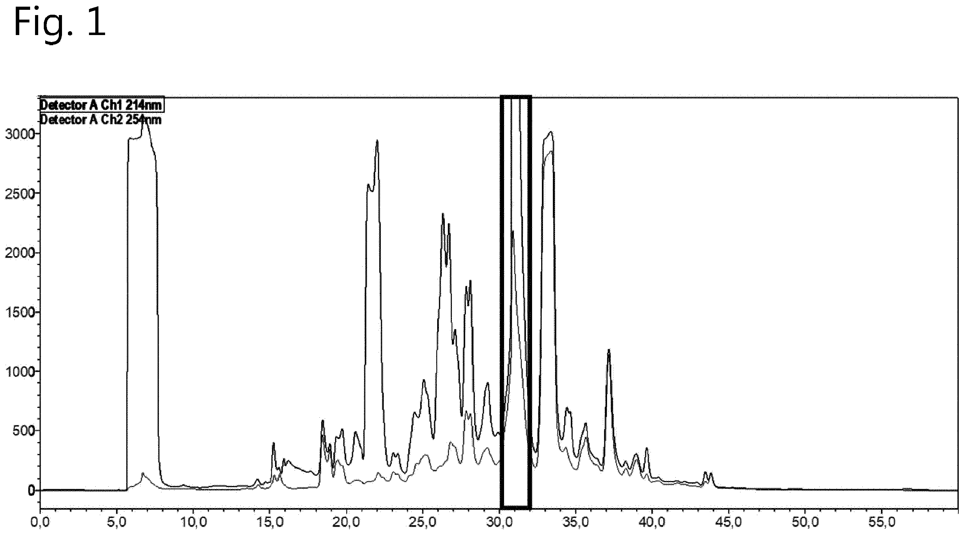

The fractions were dried in a speed-vac o/n. The fraction residues were dissolved in DMSO and tested on C. *violaceum* for quorum sensing activity in serial dilutions starting at 40x. Fraction 20 showed inhibition of violacein production in C. violaceum, which concurs with UV detection of the HPLC fractionation (Figure 1).

To purify this fraction, the remaining fraction was dried in a speed-vac o/n and dissolved in DMSO. The sample was then fractionated using aforementioned prep. HPLC. The buffers used for this purification run are described in Table 3, the buffer B gradient in table 4.

**Table 3: HPLC buffers**

| **Buffer A** | **Buffer B** |
|---|---|
| 95% MQ | 20% MQ |
| 5% Acetonitrile | 80% Acetonitrile |
| 10 mM Ammonium acetate | 10 mM Ammonium acetate |

**Table 4: HPLC gradient**

| **Time (min.)** | **Percentage buffer B** |
|---|---|
| 0-5 | 0 |
| 5-45 | 0-100 |
| 45-50 | 100 |
| 50-55 | 100-0 |
| 55-60 | 0 |

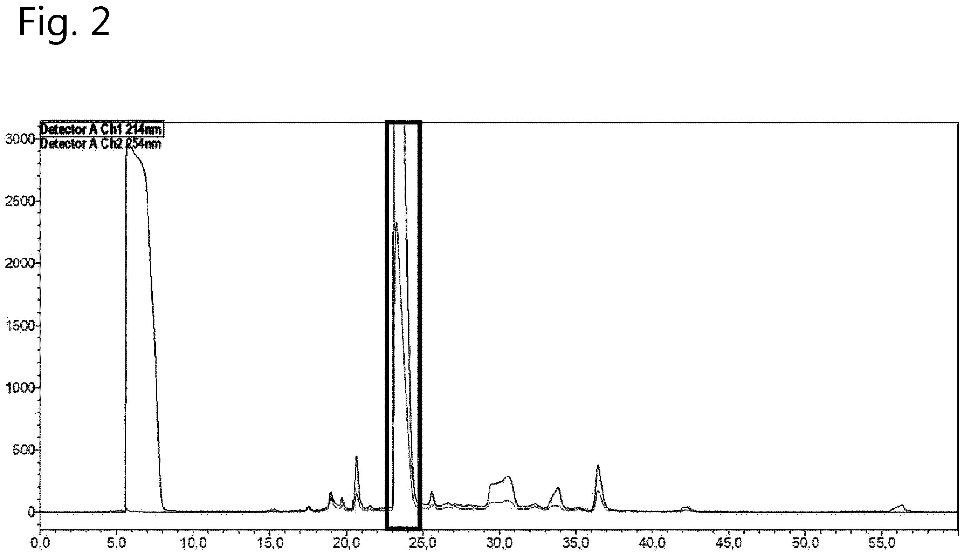

The fractions were dried in a speed-vac o/n. The fraction residues were dissolved in DMSO and tested on C. *violaceum* for quorum sensing activity in serial dilutions starting at 40x. Fraction 1 showed inhibition of violacein production in C. violaceum, which concurs with the UV detection of the HPLC fractionation (Figure 2).

### Identification of the compound with quorum sensing activity

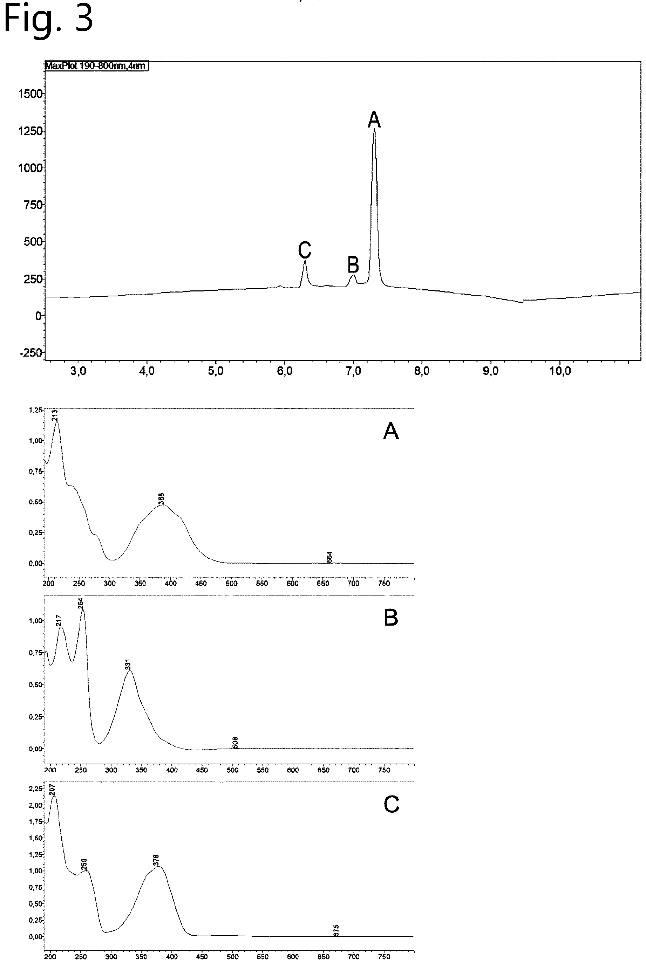

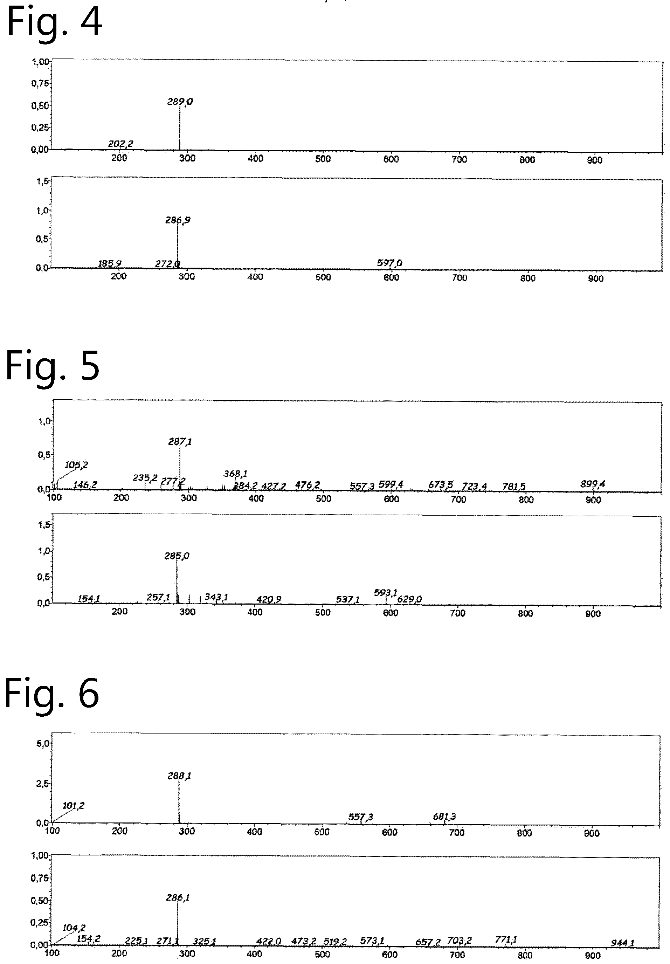

The sample was run on analytical HPLC (see Table 6 for apparatus information), which indicated the compound was 85 % pure (Figure 3). An aliquot of fraction 20.1 was analyzed using LC-MS (Figure 4) (see Table 5 for apparatus information) and high resolution mass spectrometry. The calculated mono-isotopic mass was 289.5937 with a molecular formula prediction of C₁₅H₁₂O₆. UV-VIS was also measured, revealing a characteristic UV-VIS spectrum **(213(100),** 238sh, 277sh, 388(40)) (Figure 3A). However, analytical HPLC indicated that the single fraction of the preparative HPLC resulted in three peaks with relative abundances of 85 %, 5 % and 10 % for peak A, B and C, respectively. The relative abundance of the three peaks was pH-dependent. The three peaks contain related compounds with nominal masses of 288 (peak A, Figure 3), 286 (peak B, Figure 5) and 287 (peak C, Figure 6) and UV-VIS spectrum of (217(90), **254(100),** 331(50)) (peak B, Figure 3B) and **(207(100),** 259 (50), 378 (50)) (peak C, Figure 3C).

### Elucidation of the structure of the bioactive compound

The entire fraction 20.1 was subsequently evaporated in the speed-vac and the residue was dissolved in 400µl DMSO-d6. The yield of the fraction was ~5 mg dry weight (from 3L fungal culture). A ¹H-NMR spectrum and a ¹³C-NMR spectrum were measured. Whereas not all carbons were detected, the NMR data, like the UV-VIS spectrum, showed high similarities with the data of paecilomycone, reported by Lu et al. in J. Agric. Food Chem. 2014, 62, 11917-11923. The correlations between δ_{H} 6.72 (5H) and δ_{H} 2.74 (11H₃), and between δ_{H} 6.44

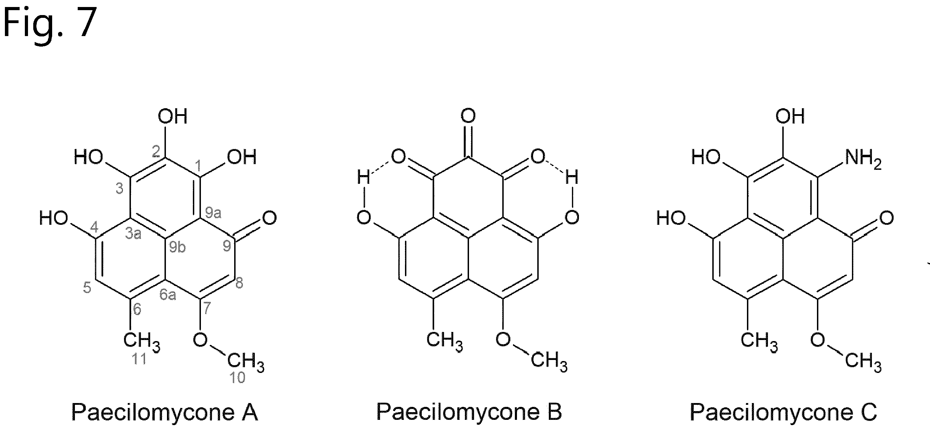

(8H) and δ_{H} 3.98 (10H₃) confirmed the position of the methoxy group. These results indicate that the peak A (Fig. 4) contains paecilomycone A. The molecular weight and UV-VIS spectra of peak B and peak C indicate that these peaks represent paecilomycone B and C, respectively (Figure 7). The various paecilomycones were not separable by HPLC and the relative concentration of each paecilomycone was pH dependent. Therefore, the combination of the three paecilomycones will be referred to as paecilomycone.

### Paecilomycone inhibits quorum sensing, biofilm formation and virulence factor production

Quorum sensing was assessed in Gram-negative C. *violaceum* bacteria. C. *violaceum* produces a purple pigment, violacein, upon activation of quorum sensing, as described in Skogman et al. A. Flavones as quorum sensing inhibitors identified by a newly optimized screening platform using chromobacterium violaceum as reporter bacteria. (2016) Molecules 21, 1211. Paecilomycone strongly inhibited violacein production with an IC₅₀ of 72.5 µM (Figure 8). Viability was also determined to distinguish between effects of paecilomycone on quorum sensing and effects on bacterial growth, which might be misinterpreted as an effect on violacein production. Toxic effects on C. *violaceum* were not detected until paecilomycone concentrations of 1mM (Figure 8). In conclusion, paecilomycone is a potent quorum sensing inhibitor in C. *violaceum* at concentrations that do not affect cell viability.

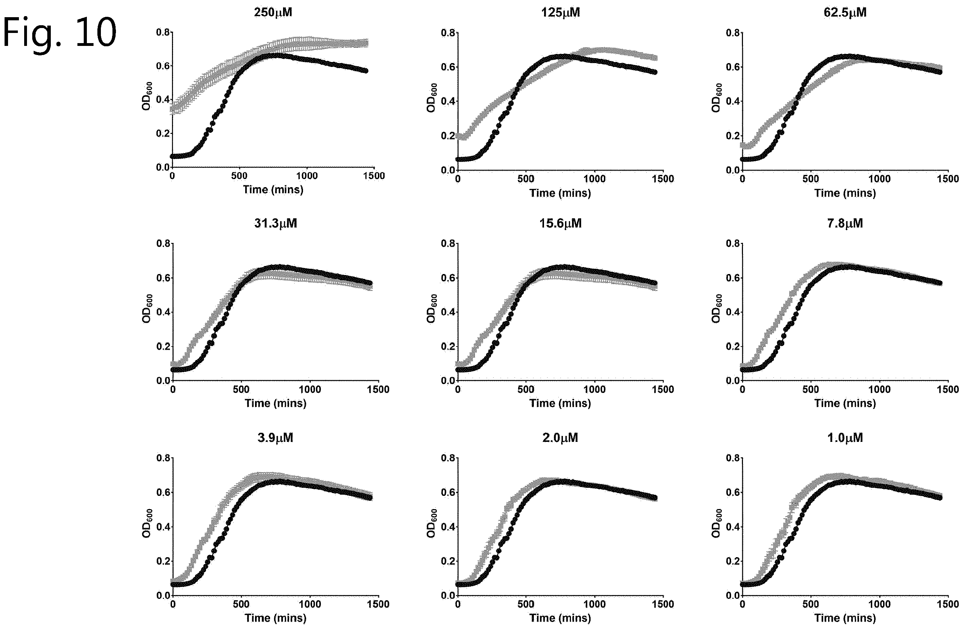

To test the potential of paecilomycone as quorum sensing inhibitor in more clinically relevant bacteria, the effect on P. *aeruginosa* (PAO1) was tested. To this end, bacterial reporter strains were used that express GFP when a quorum sensing pathway is activated, including *lasB*-GFP, rhIA-GFP, pqsA-GFP and a WT-GFP that constitutively expresses GFP as control. To correct for potential effects on bacterial growth, the OD600 was determined in parallel with GFP fluorescence intensity and RFU/OD was plotted. Paecilomycone inhibits GFP expression in the *lasB*-GFP reporter and pqsA-GFP reporter with an IC₅₀ of 49.8 µM for *lasB* and 31.5 µM for *pqsA* (Figure 9). In control WT-GFP bacteria, paecilomycone affects GFP expression with an IC₅₀ of 143.2 µM, 3- to 4.5-fold higher than in the *lasB* and *pqsA* reporters, respectively. The rhIA reporter was not sensitive to paecilomycone with an IC₅₀ of 233.8 µM, which is actually higher than of WT-GFP. These results indicate that quorum sensing is inhibited by paecilomycone in clinically relevant P. *aeruginosa* (PAO1) bacteria, irrespective of effects on bacterial growth. Treatment with high concentrations of paecilomycone (62.5 µM and higher) affected PAO1 growth rate, but not maximal bacterial density (Figure 10).

Funalenone is closely related to paecilomycone A, B and C (Figure 11A). The overall structure is very similar, but the position of the methoxy group in funalenone is different compared to paecilomycone. We tested quorum sensing inhibitor activity of commercially available funalenone (Bio-connect, Huissen, The Netherlands, AG-CN2-0137-M001) in C. *violaceum* bacteria and found that treatment with funalenone up to 1 mM did not inhibit violacein production (Figure 10B). Funalenone also did not affect cell viability in assays performed in parallel with the quorum sensing assays (Figure 11B). We also tested the effect of funalenone on quorum sensing in PAO1 reporters *lasB*-GFP, rhIA-GFP, pqsA-GFP and as a control WT-GFP and found no effects on quorum sensing at funalenone concentrations up to 500 µM (Figure 11C). We conclude that funalenone does not affect quorum sensing, which is surprising because highly related paecilomycone A, B and C did inhibit quorum sensing.

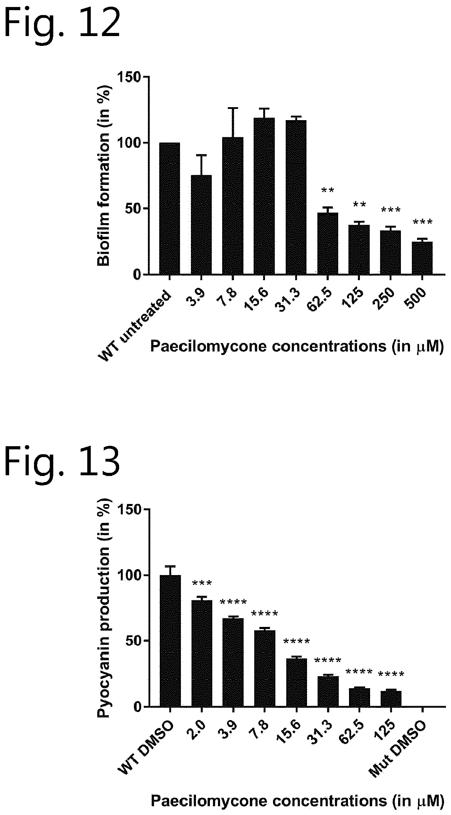

Since quorum sensing has a downstream effect on the maturation of biofilm in P. *aeruginosa,* as described in Jakobsen et al. Bacterial biofilm control by perturbation of bacterial signaling processes. (2017) Int. J. Mol. Sci. 18, 1970, we tested the effect of paecilomycone on biofilm formation of WT PAO1 bacteria. Paecilomycone has a significant inhibitory effect on biofilm formation from 62.5 µM onwards (Figure 12). It is unlikely that this effect is due the effect on bacterial growth. Whereas bacterial growth rate is reduced in the presence of 62.5 µM paecilomycone or more, the bacterial density after 24 h is indistinguishable between treated and untreated bacteria. These results indicate that paecilomycone affects biofilm formation in PAO1 bacteria by a mechanism other than merely affecting bacterial growth.

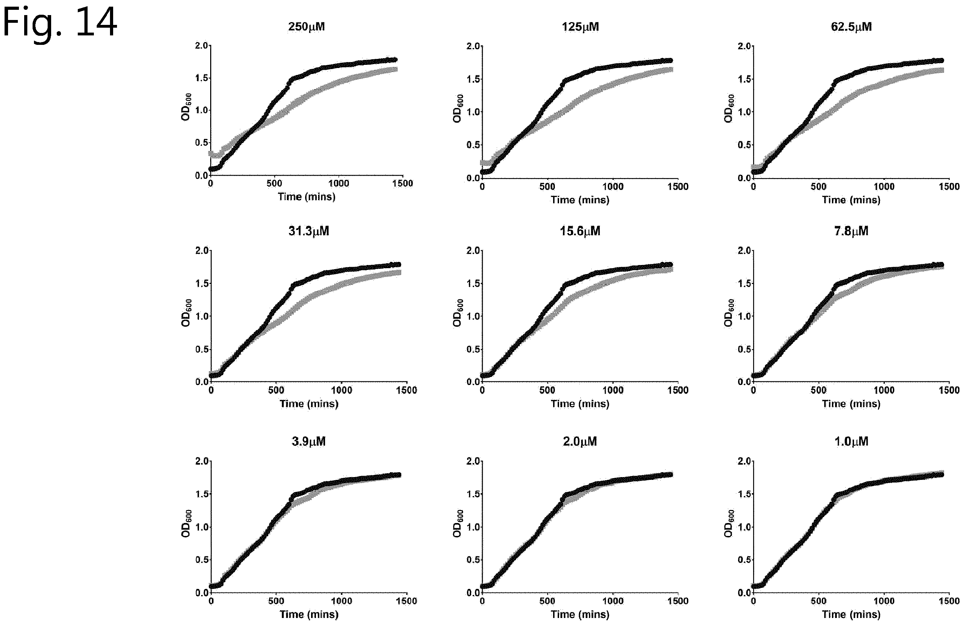

Another downstream effect of quorum sensing in *P. aeruginosa* is the production of virulence factors, as described in Rutherford & Bassler. Bacterial quorum sensing: Its role in virulence and possibilities for its control. (2012) Cold Spring Harb. Perspect. Med. 2, 1-25. The effect of paecilomycone on various virulence factors was assessed. Paecilomycone induced a strong and significant inhibitory effect on the production of pyocyanin with a maximum inhibition of 88 % and an IC₅₀ of 8.5 µM. The lowest concentration tested, 1.95 µM, already showed a significant decrease of pyocyanin production (Figure 13). Pyocyanin production was determined in King's A medium. To rule out inadvertent effects on PAO1 growth due to the medium, growth kinetics were also determined in King's A medium. The initial slopes of the PAO1 bacterial density curves were similar in control and paecilomycone treated samples, indicating bacterial growth is similar (Figure 14). Yet, bacterial density did not reach the same level in samples treated with high paecilomycone concentrations (from 31.3 µM onwards). However, the difference in bacterial densities after 24 h treatment were relatively small and could not explain the large decrease in pyocyanin production in response to paecilomycone (cf. Figure 13 and 14). These results indicate that pyocyanin production was significantly affected in PAO1 bacteria in response to paecilomycone treatment.

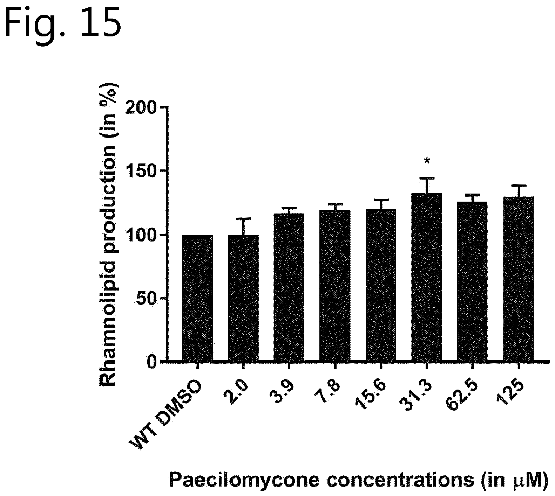

Rhamnolipid production in PAO1 cells in response to paecilomycone was increased. The increase was modest, up to 33 % and the variation between individual samples of the triplicate measurements was large (Figure 15). Only following treatment with 31 µM of paecilomycone, the effect was statistically significant. These results indicate that paecilomycone induced a modest increase in rhamnolipid production in PAO1 bacteria.

### Growth kinetics of bacteria in the presence of the paecilomycone

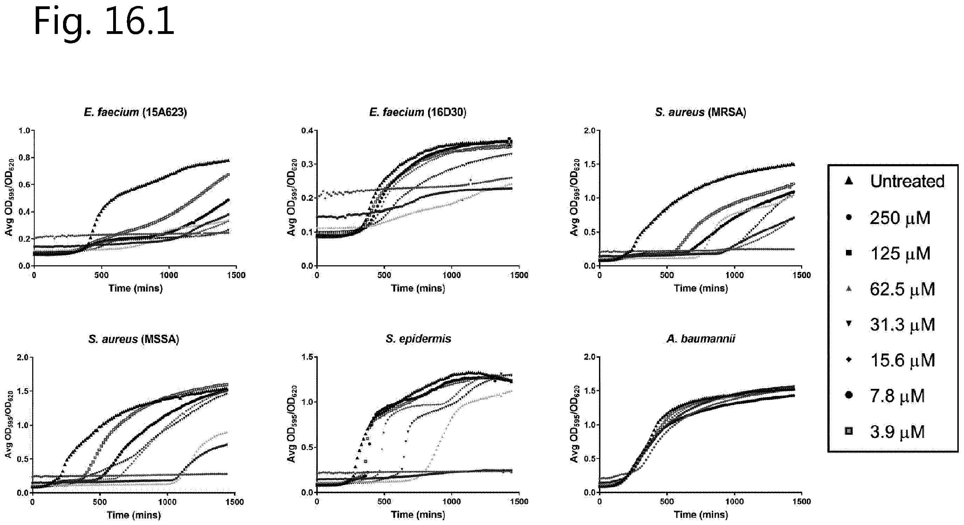

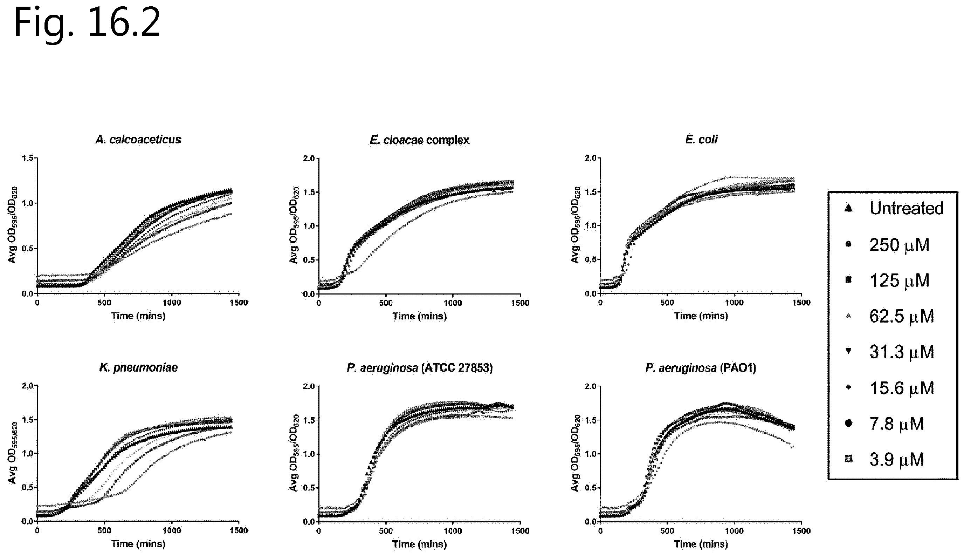

After elucidating the structure and establishing quorum sensing inhibitory activity of paecilomycone, we studied the growth kinetics of several bacterial strains in the presence of 1, 2, 4, 8, 16, 32, 63, 125 or 250 µM paecilomycone, in order to determine whether the compound had antibacterial activity. The bacteria were cultured at 37°C for 24 h in the presence of the compound and the OD600 was determined every 15 min for 24 h. The bacteria tested were two strains of *Enterococcus faecium* (15A623 and 16D30), MRSA USA300, MSSA, *Staphylococcus epidermidis, A. baumanii, A. calcoaceticus, E. cloacae* complex, *E.coli*, *K. pneumoniae*, *P.aeruginosa* (ATCC 27853) and *P.aeruginosa* (PAO1) (Figure 16). Based on these growth curves, we can conclude that paecilomycone *inhibits growth of Enterococcus faecium* (15A623 and 16D30), MRSA USA300, MSSA, *Staphylococcus epidermidis and K. pneumoniae* in MHB medium.

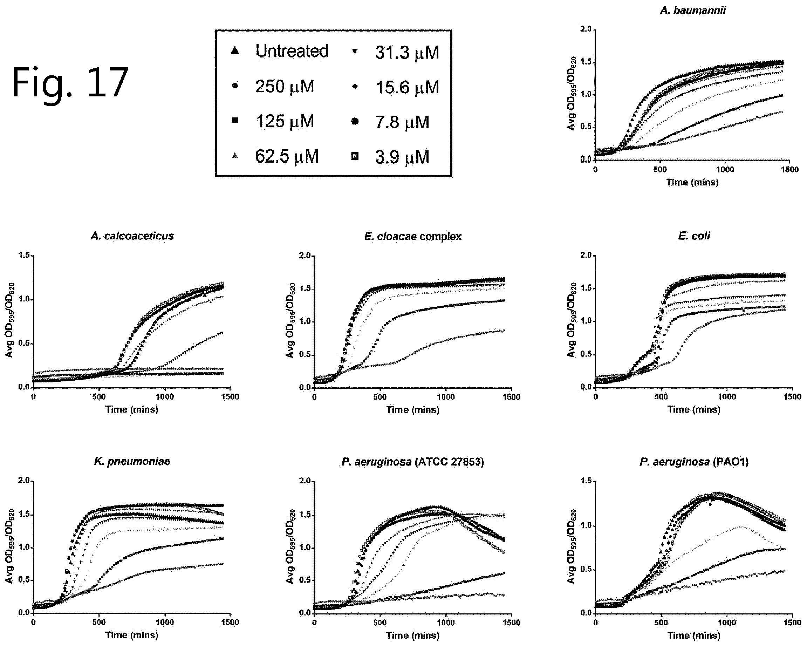

A similar assay was performed, growing the bacteria in AB minimal medium. The bacteria tested in this medium were A. *baumanii*, *A. calcoaceticus, E. cloacae* complex, *E.coli*, *K. pneumoniae*, *P.aeruginosa* (ATCC 27853) and *P.aeruginosa* (PAO1) (Figure 17).

Based on these growth curves, we can conclude that paecilomycone affects growth of *A. baumanii*, *A. calcoaceticus*, *E. cloacae* complex, *E.coli*, *K. pneumoniae*, *P.aeruginosa* (ATCC 27853) and *P.aeruginosa* (PAO1) in AB minimal medium.

### Cytotoxicity

Cytotoxicity of paecilomycone on eukaryotic cells was tested using human liver-derived HepG2 cells. Viability of HepG2 cells was assessed following treatment with a range of concentrations of paecilomycone for 24 h. At high concentrations, paecilomycone was toxic to HepG2 cells with an IC₅₀ of 219 µM (Figure 184). Up to 125 µM paecilomycone, there is no difference in the viability of HepG2 cells compared to untreated control cells. These results show that paecilomycone is not cytotoxic to human cells at concentrations that effectively inhibit quorum sensing and/or inhibit bacterial growth, indicating there is a concentration window for treatment of bacterial infections.

**Table 5: Apparatus**

| | **Apparatus** | **Column** |
|---|---|---|
| **Prep. HPLC** | Shimadzu System Controller: CBM-20A controllerPiston pump: Shimadzu LC-20AP | Dr. Maisch Reversed phase C18 Reprosil-Pur 120 C18-AQ Particle size: 10 µm |
| | | Pore size: 120 Å |
| | UV-VIS detector: Shimadzu SPD-20A (standard 214nm and 254nm) | Carbon load: 15 % |
| | | pH range: 1-10 |
| | Fraction collector: Shimadzu FRC-10A | |
| **Analytical HPLC** | LC-2030C 3D Plus | Dr. Maisch Reprosil-PUR 120 C18 AQ; 100x4.6mm; |
| | System controller: SCL-10A | |
| | Pump: LC-10AT | Particle size: 3 µm |
| | UV-VIS detector: SPD-10A (standard 214nm and 254nm) | Pore size: 120 Å |
| | | Carbon load: 15 % |
| | Auto injector: SIL-10AD (sample loop 50µl) | pH range: 2-8 |
| **LC-MS** | LC-2030C 3D PlusColumn oven: CTO-10AS | Dr. Maisch Reprosil-Pur 120 C18 AQ; 100x4.6mm; |
| | System controller: SCL-10A | Particle size: 3 µm |
| | Pump: LC-10AD | Pore size: 120 Å |
| | UV-VIS detector: SPD-10A (standard 214nm and 254nm) | Carbon load: 15 % |
| | | pH range: 2-8 |
| | Auto injector: SIL-10AD (sample loop 50µl) | |
| | MS: LCMS-2020 | |

## Claims

1. A compound of structure (1): 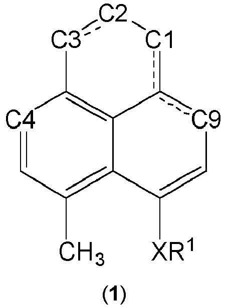 wherein:
- each of C1, C2, C3, C4 and C9 are individually selected from C=O, C-OH, C-OR², C=S, C-SH, C-SR², C-NH₂ or C-N(R²)₂, wherein the carbon atom is part of the phenalene ring structure, and wherein each R is individually C₁₋₆ alkyl, C(Y)-C₁₋₆ alkyl, C(Y)-Y-C₁₋₆ alkyl and C(Y)-N(H)₀₋₂(C₁₋₆ alkyl)₀₋₂, wherein each Y is individually O or S;
- R¹ is selected from monocyclic aryl, C₁₋₆ alkyl, C(O)-C₁₋₆ alkyl, C(O)-O-C₁₋₆, alkyl and C(O)-N(H)₀₋₂(C₁₋₆, alkyl)₀₋₂;
- X is S or O,
for use in treating or preventing a microbial infection.

2. The compound for use according to claim 1, wherein C1, C2, C3, C4 and C9 are individually selected from C=O, C-OH or C-NH₂.

3. The compound for use according to claim 1 or 2, wherein there are an odd number of C=O moieties, preferably 1 or 3.

4. The compound for use according to any one of claims 1 - 3, wherein one of C1 and C9 is C=O, and/or C2 and C3 are both C=O or are both not C=O.

5. The compound for use according to any one of claims 1 - 4, wherein the compound is selected from paecilomycone A, paecilomycone B, paecilomycone C and mixtures thereof.

6. The compound for use according to any one of claims 1 - 5, wherein XR¹ is OCH₃.

7. The compound for use according to any one of claims 1 - 6, wherein the microbial infection is a bacterial infection.

8. The compound for use according to claim 7, wherein the bacterial infection is an infection by a multidrug resistant bacterium.

9. The compound for use according to claim 7 or 8, wherein the bacterial infection is an infection by a Gram-positive bacterium or a Gram-negative bacterium, preferably a Gram-positive bacterium.

10. The compound for use according to any one of claims 1 - 9, wherein the treatment is a combination treatment of the compound of structure (1) together with a second active component, preferably wherein the second active component is an antibiotic.

11. Use of a compound as defined in any one of claims 1 - 6 for preventing or reducing biofilm formation.

12. Use of a compound as defined in any one of claims 1 - 6 for preventing or reducing bacterial proliferation.

13. Use of a compound as defined in any one of claims 1 - 6 for inhibiting bacterial quorum sensing, biofilm formation and virulence factor production.

14. Use of a compound as defined in any one of claims 1 - 6 as antimicrobial agent.

15. Antimicrobial composition, comprising a compound as defined in any one of claims 1 - 6.
